# EUROPEAN PATENT APPLICATION

(11) **EP 4 248 911 A1**
(43) Date of publication of application: **27.09.2023**
(21) Application number: 23155749.7
(22) Date of filing: 09.02.2023
(51) Int. Cl.: A61F 2/06, A61F 2/856

(54) **ENDOVASCULAR STENT GRAFT HAVING GATE AND IMPLANT JOINING LINER**

(30) Priority: 17.02.2022 US 202263311237 P; 19.01.2023 US 202318099018
(71) Applicant: Medtronic Vascular Inc., Santa Rosa, CA 95403 (US)
(72) Inventor: SHIPLEY, Adam J., Santa Rosa, 95403 (US)
(74) Representative: Zimmermann & Partner Patentanwälte mbB

(57) **Abstract**

An endovascular stent graft is provided. The endovascular stent graft includes a body including a gate having an internal surface, a joining liner joined to the internal surface of the gate with a joint and having a bunched state and a crumpled state, and an implant at least partially disposed within the joining liner and having a radially compressed state and a radially expanded state. The implant in the radially expanded state exerts a radial force on the joining liner to maintain the joining liner in the crumpled state.

## Description

### CROSS-REFERENCE TO RELATED APPLICATION

This application claims the benefit of U.S. provisional application Serial No. 63/311,237 filed February 17, 2022, the disclosure of which is hereby incorporated in its entirety by reference.

### TECHNICAL FIELD

The present disclosure relates generally to an endovascular stent graft (e.g., a branched stent graft) having a gate with a joining liner joining an implant to the gate. The joining liner may be joined to an internal surface of the gate.

### BACKGROUND

Endovascular procedures are minimally invasive techniques delivering clinical treatments and repairs to a patient's vasculature. A stent graft is an implantable device used in endovascular procedures. The stent graft includes a tube-shaped surgical graft and an expanding stent frame. The stent graft may be placed inside a blood vessel to bridge a diseased segment of the blood vessel (e.g., an aneurismal, dissected, or torn segment of the blood vessel). The stent graft is configured to exclude hemodynamic pressures of blood flow from the diseased segment of the blood vessel.

Depending on the region of the aorta involved, the diseased segment may extend into vessel bifurcations or segments (otherwise referred to as branches) of the aorta. Thoracic aortic aneurysms are examples of diseased segments that may present in the ascending thoracic aorta, the aortic arch, and/or branch arteries (e.g., left subclavian, left common carotid, or the brachiocephalic arteries). In some cases, a branched stent graft can be used to treat such aneurysms. For example, a branched stent graft can be deployed in the main vessel (e.g., aortic arch) with a branch extending therefrom and toward or into the branched artery (e.g., left subclavian), and a supplemental, secondary stent graft can be deployed in the branched artery and connected to the branch. In other applications, a branched stent graft may also include branch and tributary legs for extending the branched stent graft into other blood vessels branching from the aorta (e.g., renal, celiac, or mesenteric arteries).

### SUMMARY

According to one embodiment, an endovascular stent graft is disclosed. The endovascular stent graft includes a body including a gate having an internal surface, a joining liner joined to the internal surface of the gate with a joint and having a bunched state and a crumpled state, and an implant at least partially disposed within the joining liner and having a radially compressed state and a radially expanded state. The implant in the radially expanded state exerts a radial force on the joining liner to maintain the joining liner in the crumpled state.

According to another embodiment, an endovascular stent graft is disclosed. The endovascular stent graft includes a body including a gate having an internal surface and a joining liner joined to the internal surface of the gate with a distal joint and a proximal joint. At least one of the distal and proximal joints extend around at least one of distal and proximal circumferences, respectively, of the gate. The endovascular stent graft also includes an implant at least partially disposed within the joining liner and having a radially compressed state and a radially expanded state. The implant in the radially expanded state contacts the joining liner.

According to yet another embodiment, a method for joining an implant with a gate of an endovascular stent graft is disclosed. The method includes the step of inserting the implant in a radially compressed state into the gate having an internal joining liner having a bunched state and a crumpled state. The method further includes translating the implant from the radially compressed state into a radially expanded state to exert a radial force on the joining liner to maintain the joining liner in the crumpled state and to form a j oint between the implant and the joining liner of the gate.

### BRIEF DESCRIPTION OF THE DRAWINGS

Figure 1A is a side view of a branched stent graft according to a first embodiment.
Figure 1B is a partial cross-section view of a portion of a gate of the branched stent graft of Figure 1A taken along line 1B-1B and showing a joining liner according to the first embodiment.
Figure 1C is a cross-section view of a portion of a gate of a branched stent graft showing a joining liner with a parabolic shaping characteristic.
Figure 1D is a cross-section view of a portion of a gate of a branched stent graft showing a joining liner with a tapered shaping characteristic.
Figure 1E is a partial cross-section view showing an implant and a gate joined using a joining liner according to the first embodiment.
Figure 1F is a top view of an opening of a gate including a radially bunched liner according to one embodiment.
Figure 2A is a side view of a branched stent graft according to a second embodiment.
Figure 2B is a partial cross-section view of a portion of a gate of the branched stent graft of Figure 2A taken along line 2B-2B showing a joining liner according to the second embodiment.
Figure 2C is a partial cross-section view of a portion of the gate of the branched stent graft of Figure 2A taken along line 2C-2C showing an alternative joining liner according to another embodiment.
Figure 2D is a partial cross-section view showing an implant and a gate joined using a joining liner according to the second embodiment.
Figure 2E is a cross-section view of a tapered branching gate and a tapered implant having aligned proximal ends.
Figure 2F is a cross-section view of the tapered branching gate and the tapered implant having unaligned proximal ends.
Figure 3 is a partial cross-section view of a portion of a gate of a branched stent graft where the material of the gate is folded inward back on itself to form a joining liner according to another embodiment.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. Each embodiment and each aspect so defined may be combined with any other embodiment or with any other aspect unless clearly indicated to the contrary. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

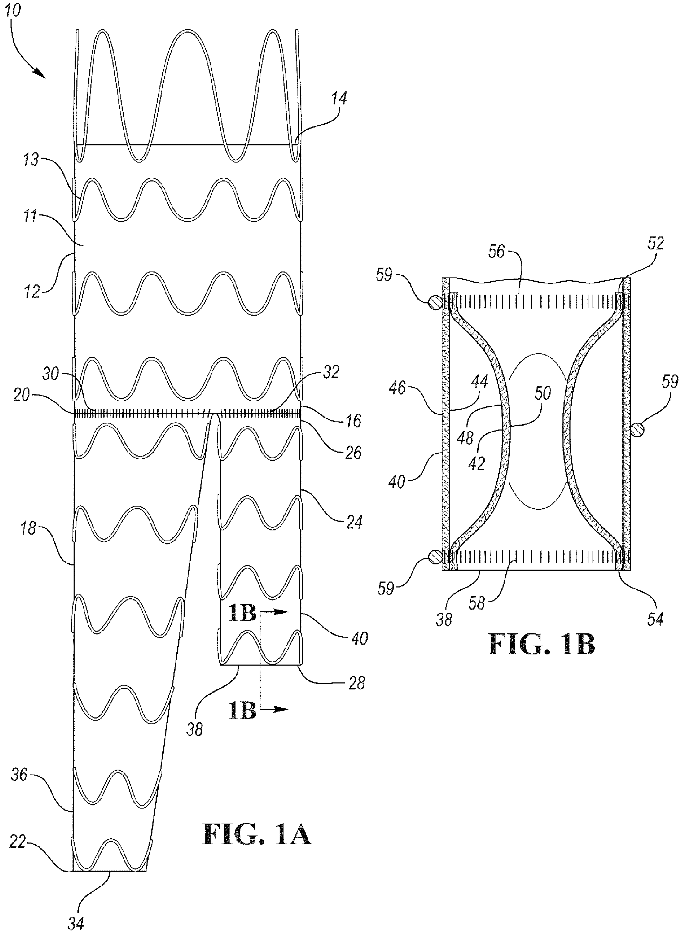

Figure 1A is a side view of branched stent graft 10 according to a first embodiment. Branched stent graft 10 includes graft material 11 secured to one or more stents 13. Graft material 11 of branched stent graft 10 may be formed of a semi-permeable material or non-permeable material. A non-limiting example of a semi-permeable material is woven polyester terephthalate (PET). Non-limiting examples of non-permeable material include polyester terephthalate (PET), expanded polyester terephthalate (ePET), polytetrafluoroethylene (PTFE), and combinations thereof.

Branched stent graft 10 includes main body 12 extending between proximal end 14 and distal end 16. Branched stent graft 10 also includes first leg 18 extending between proximal end 20 and distal end 22, and second leg 24 extending between proximal end 26 and distal end 28. Proximal end 20 of first leg 18 is joined with distal end 16 of main body 12 with first seam 30. Proximal end 26 of second leg 24 is joined with distal end 16 of main body 12 with second seam 32. First leg 18 defines first opening 34 at first gate 36, which refers to a distal portion of first leg 18. Second leg 24 defines second opening 38 at second gate 40, which refers to a distal portion of second leg 24. Second gate 40 may be a contralateral gate and branched stent graft 10 may be a bifurcated stent graft.

Figure 1B is a partial cross-sectional view of a portion of second gate 40 of branched stent graft 10 taken along line 1B-1B of Figure 1A. Figure 1B shows joining liner 42 according to the first embodiment. Joining liner 42 may have a tubular shape. Second gate 40 extends radially between inner surface 44 and outer surface 46 of second gate 40. Joining liner 42 extends radially between outer surface 48 and inner surface 50 of joining liner 42. Joining liner 42 extends axially from proximal end 52 to distal end 54. Proximal end 52 of joining liner 42 is joined to inner surface 44 of second gate 40 with proximal joint 56. Distal end 54 of joining liner 42 is joined to inner surface 44 of second gate 40 with distal joint 58. Upon forming proximal and distal joints 56 and 58, outer surface 48 of joining liner 42 faces inner surface 44 of second gate 40. A similar joining liner may be formed in first gate 36.

Joining liner 42 may be formed of a semi-permeable material or a non-permeable material. A non-limiting example of a semi-permeable material is woven polyester terephthalate (PET). Non-limiting examples of non-permeable material include polyester terephthalate (PET), expanded polyester terephthalate (ePET), polytetrafluoroethylene (PTFE), and combinations thereof. Joining liner 42 may be a woven textile material. Joining liner 42 may be formed of a polymeric material using a forming process (e.g., extrusion process or an electro-spinning process). The material forming joining liner 42 may be the same or may be different than the material forming graft material 11 of branched stent graft 10. The material of joining liner 42 may have different material properties (e.g., greater flexibility and/or less strength) than the material forming second gate 40 because of the different functions performed by the two materials. The material of joining liner 42 may be configured to reduce or eliminate leak paths between implant 60 while the material of second gate 40 may be stronger to exclude hemodynamic pressures of blood flow from a diseased segment of a blood vessel. In one or more embodiments, the material forming joining liner 42 may be less expensive than the material forming second gate 42.

Proximal and/or distal joints 56 and/or 58 may be formed of stitching and/or sutures formed of a textile material or a polymeric material. In another embodiment, proximal and/or distal joints 56 and/or 58 may be formed of a melted and solidified mixture of a portion of graft material 11 of branched stent graft 10 and joining liner 42. The melted/solidified mixture joint may be applicable when joining liner 42 is formed of a polymeric material. The joints may also be formed of adhesives, staples, or any other joining mechanism. In one or more embodiments, both joints may extend continuously around the entire circumference of second gate 40 to create a seal between joining liner 42 and the graft material of second gate 40. In other embodiments, only one of the joints extend continuously around the entire circumference of second gate 40.

As shown in Figure 1B, joining liner 42 is bunched such that a medial portion of joining liner 42 extends inwardly toward the longitudinal axis of second gate 40. Figure 1B shows joining liner 42 in an axially bunched state. The axial length of joining liner 42 between first and second joints 56 and 58 is greater than the axial length of second gate 36 between first and second joints 56 and 58 to produce the bunching and billowing in as shown in Figure 1B. The axial length of joining liner 42 may be greater than the axial length of second gate by any of the following percentages or in a range of any two of the following percentages: 5%, 10%, 20%, 25%, 50%, 75%, 100%, 125%, 150%, 175%, 200%, 225%, 250%, 275%, or 300%. As shown in Figure 1B, first and/or second joints 56 and/or 58 are aligned with one or more stents 59 of stents 13 to reinforce the strength of first and/or second joints 56 and/or 58.

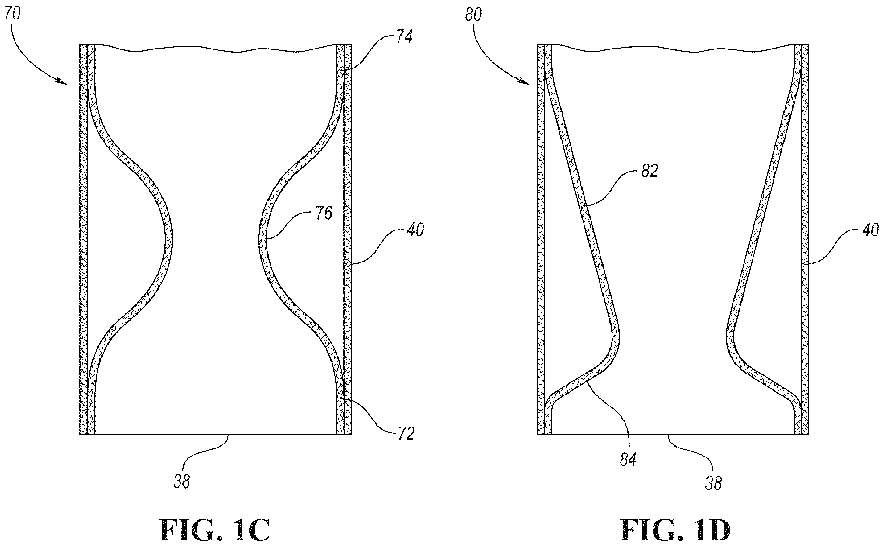

As shown in Figure 1B, joining liner 42 has a curved shape between first and second joints 56 and 58. The joining liner may have different shapes than the shape shown in Figure 1B. For example, Figure 1C shows joining liner 70 having first and second flat portions 72 and 74 with curved portion 76 extending therebetween. As shown in Figure 1C, curved portion 76 is parabolic and symmetrical. In other embodiments, the curved portion is non-symmetrical. As a further example, Figure 1D shows joining liner 80 having first and second tapered portions 82 and 84. As shown in Figure 1D, tapered portion 82 is longer than tapered portion 84 and the angle of tapered portion 82 relative to second gate 40 is less than the angle of tapered portion 84 relative to second gate 40. In other embodiments, the angle of tapered portion 82 relative to second gate 40 is greater than the angle of tapered portion 84 relative to second gate 40. The shaping characteristics of the joining liner may be selected based on the type of implant inserted into the gate. The bunching shown in Figure 1B may be a result of the dimensions of the joining liner before it is joined to the interior surface of the gate. In other words, the joining liner may not further bunch upon being joined to the interior surface of the gate. The bunching shown in Figures 1C and 1D may be a result of excess material formed by a relatively larger diameter in the region of bunching. The material used in the embodiments shown in Figures 1C and 1D may be expandable (e.g., elastic polymer or a woven textile) such that the bunched region expands upon insertion of an implant.

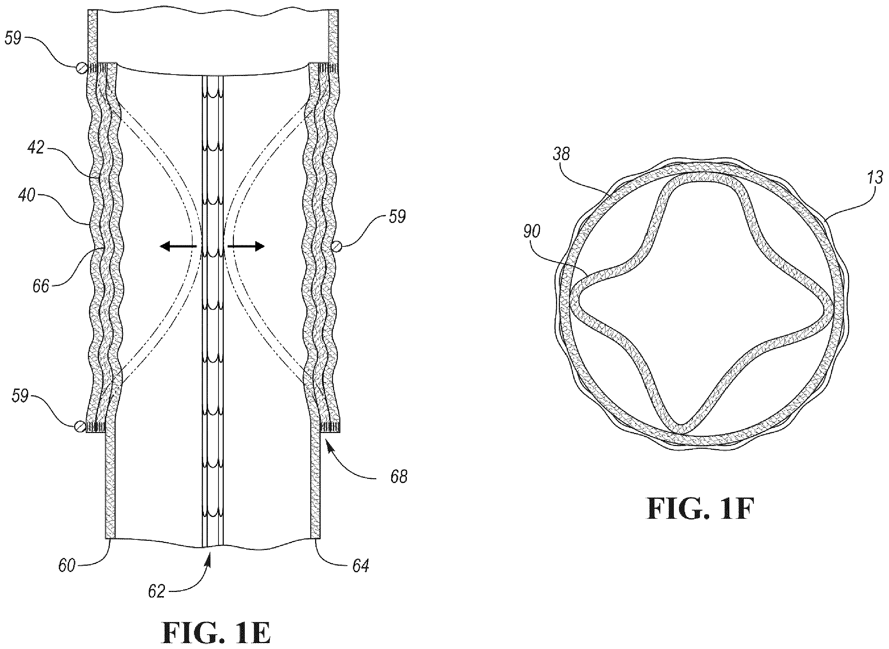

Figure 1E is a partial cross-section view showing implant 60 and second gate 40 joined using joining liner 42 according to the first embodiment. Implant 60 is inserted partially within second gate 40 in radially compressed state 62. During a deployment process, implant 60 transitions from radially compressed state 62 to radially expanded state 64, thereby exerting a radial outward force against joining liner 42 to transition joining liner 42 from the axially bunched state shown in Figures 1B and 1E to crumpled state 66 as shown in Figure 1E. During the deployment process, joining liner 42 in the axially bunched state may partially occlude implant 60 until implant 60 transitions from radially compressed state 62 to radially expanded state 64 without significant reduction in the volume of lumen of second gate 40 post deployment.

Joining liner 42 is configured to reduce leakage through joint 68 formed between implant 60 and second gate 40 when joining liner 42 is in crumpled state 66 and implant 60 is in radially expanded state 64. Joining liner 42 in crumpled state 66 does not significantly reduce volume of the lumen formed by second gate 40 such that the flow of blood or other fluids is not significantly reduced after the implant 60 is deployed within second gate 40. Joining liner 42 is configured to increase the strength and migration resistance of joint 68 between second gate 40 and implant 60. In one or more embodiments, the structural integrity of joining liner 42 may be less than the structural integrity of the graft material of second gate 40 because the primary function of joining liner 42 is a filler to reduce leakage paths, increase joint strength, and/or increase migration resistance whereas the graft material of second gate 40 imparts structural integrity to branched stent graft 10.

Joining liner 42 may improve endurance reliability by creating a buffer between implant 60 and second gate 42 to achieve leakage reductions, strength increases, and/or migration resistance without additional attachment mechanisms, stents, and/or sponges. Joining liner 42 may also reduce packaging density using a lower cost material with less structural integrity as opposed to additional attachment mechanisms, stents, and/or sponges.

In one or more embodiments, the joining liner may be bunched along a circumference of the gate. Figure 1F depicts second opening 38 of second gate 40 including joining liner 90. The length of joining liner 90 at a circumference of second gate 40 taken at a longitudinal location thereof is greater than the circumference of second gate 40 taken at the longitudinal location. In the embodiment shown in Figure 1F, the greater length of joining liner 90 produces a radially bunched state configured to be crumpled by an implant to produce a crumpled state, thereby reducing leakage through the joint. The material used in the embodiments shown in Figures 1F may be expandable (e.g., elastic polymer or a woven textile) such that the bunched region expands upon insertion of an implant. The joining liner may be axially and/or radially bunched (e.g., having axially and/or radially bunched states) depending on how the joining liner is applied to reduce leakage through the joint.

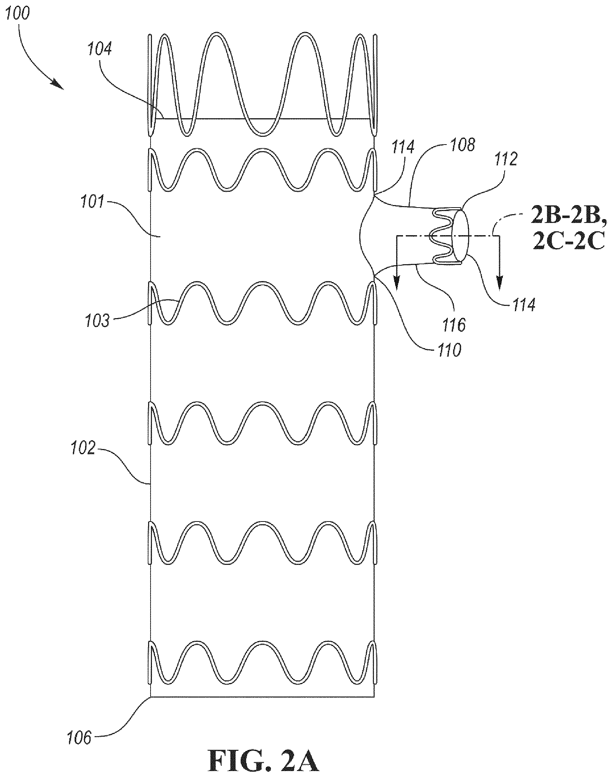

Figure 2A is a side view of branched stent graft 100 according to a second embodiment. Branched stent graft 100 includes graft material 101 secured to one or more stents 103. Graft material 101 of branched stent graft 100 may be formed of a non-permeable material, such as the non-permeable materials disclosed herein.

Branched stent graft 100 includes main body 102 extending between proximal end 104 and distal end 106. Branched stent graft 100 also includes coupling 108. Coupling 108 may be a branching gate of a thoracic stent graft (e.g., branched stent graft 100). Coupling 108 may be positioned such that when branched stent graft 100 is deployed, coupling 108 is aligned with and extends into the left subclavian artery. In other embodiments, coupling 108 may be located on branched stent graft 100 to align with and extend into other branches of the aorta such as the brachiocephalic artery, the left common carotid artery, a renal artery, the celiac artery, or the SMA. Coupling 108 tapers inwardly from wide proximal end 110 to narrow distal end 112. Coupling 108 defines opening 114 at branching gate 116, which refers to a distal portion of coupling 108.

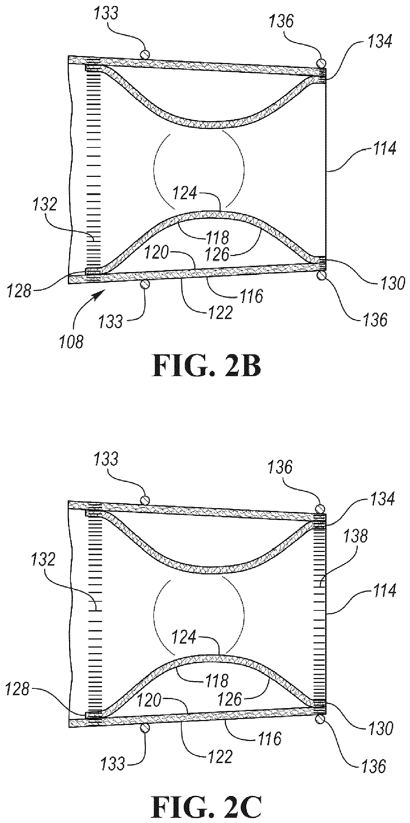

Figure 2B is a partial cross-sectional view of a portion of branching gate 116 of coupling 108 taken along line 2B-2B of Figure 2A. Figure 2B shows joining liner 118 according to the second embodiment. Branching gate 116 extends radially between inner surface 120 and outer surface 122. As shown in Figure 2B, branching gate 116 had a conical shape. Joining liner 118 may also have a conical shape complimentary to the conical shape of branching gate 116. Joining liner 118 extends radially between inner surface 124 and outer surface 126 of joining liner 118. Joining liner 118 extends axially from proximal end 128 to distal end 130 of joining liner. Proximal end 128 of joining liner 118 is joined to inner surface 120 of branching gate 116 with proximal joint 132. Distal end 134 of joining liner 118 is joined to inner surface 120 of branching gate 116 with distal joint 134. Upon forming proximal and distal joints 132 and 134, outer surface 126 of joining liner 118 faces inner surface 120 of branching gate 116. Joining liner 118 may be formed of the materials set forth herein regarding other embodiments.

Proximal joint 132 may be formed of stitching and/or sutures formed of a textile material or a polymeric material. In another embodiment, proximal joint may be formed of a melted and solidified mixture of a portion of graft material 101 of branched stent graft 100 and joining liner 118. The joints may also be formed of adhesives, staples, or other joining mechanisms. As shown in Figure 2B, proximal stent portions 133 of stents 103 are offset proximal joint 132 in a distal, axial direction. Proximal joint 132 may extend continuously around the entire circumference of branched gate 116.

As shown in Figure 2B, distal joint 134 is formed as part of the joint joining distal stent portions 136 of stents 103 with the graft material of branching gate 116. In the embodiment shown in Figure 2B, the strength of distal joint 134 is reinforced by distal stent portions 136 of stents 103. As shown in Figure 2C, alternative distal joint 138 extends continuously around the entire circumference of branched gate 116. In one alternative, the joint between distal stent portions 136 and graft material 101 forms a portion of distal joint 138. In another alternative, the joint between distal stent portions 136 and graft material 101 are separate from distal joint 138 between branching gate 116 and joining liner 118.

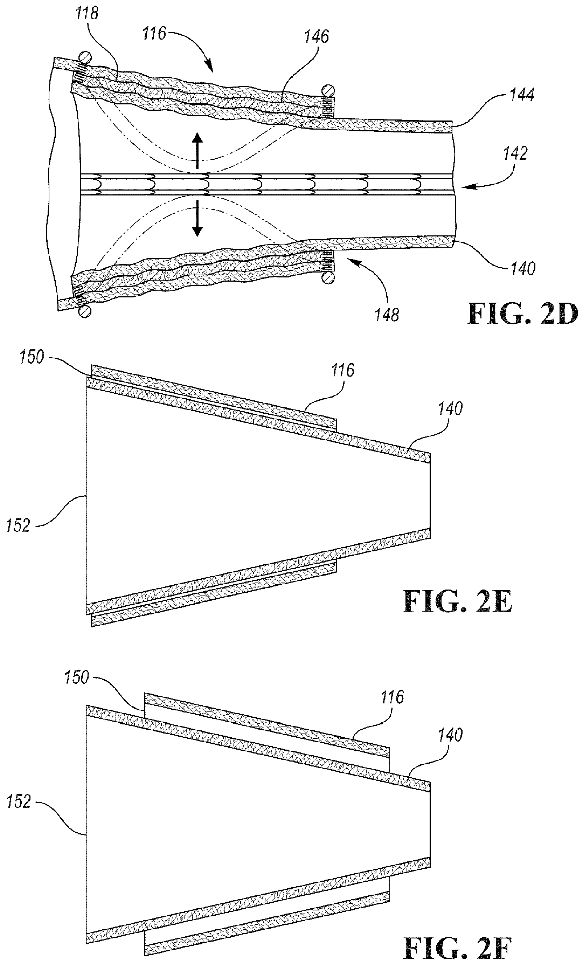

Figure 2D is a partial cross-section view showing implant 140 and branching gate 116 joined using joining liner 118 according to the second embodiment. Implant 140 is inserted partially within branching gate 116 in radially compressed state 142. During a deployment process, implant 140 transitions from radially compressed state 142 to radially expanded state 144, thereby exerting a radial outward force against joining liner 118 to transition joining liner 118 from the axially bunched state shown in Figures 2B, 2C, and 2D to crumpled state 146 as shown in Figure 2D. During the deployment process, joining liner 116 in the axially bunched state may partially occlude implant 140 until implant 140 transitions from radially compressed state 142 to radially expanded state 144 without significant reduction in the volume of lumen of branching gate 116 post deployment.

Joining liner 118 is configured to reduce leakage through joint 148 formed between implant 140 and branching gate 116 when joining liner 118 is in crumpled state 146 and implant 140 is in radially expanded state 64. When branching gate 116 and implant 140 have tapered shapes (e.g., conical shapes), inaccuracy in deployment (e.g., proximal deployment) of implant 140 within branching gate 116 may result in additional leakage paths reducing seal performance. Joining liner 118 is beneficial in reducing or eliminating such leakage paths. Utilizing a joining liner such as joining liner 118 enables proper alignment between the tapered shapes of branching gate 116 and implant 140 such that proximal ends 150 and 152 of branching gate 116 and implant 140, respectively, align as shown in Figure 2E. This alignment reduces and may prevent leakage between branching gate 116 and implant 140. As shown in Figure 2F, the proximal ends 150 and 152 of branching gate 116 and implant 140, respectively, are not aligned, thereby creating a gap 154 between branching gate 116 and implant 140. Gap 154 may cause leakage, which is reduced and may be eliminated by using a joining liner when the deployment is not entirely accurate to provide acceptable or no leakage even if alignment is not perfect. The joining liner may stop the proximal end of the implant from extending beyond the proximal end of the branching gate.

Joining liner 118 in crumpled state 146 does not significantly reduce volume of the lumen formed by branching gate 116 such that the flow of blood or other fluids is not significantly reduced after the implant 140 is deployed within branching gate 116. Joining liner 118 is configured to increase the strength and migration resistance of joint 148 between branching gate 116 and implant 140. In one or more embodiments, the structural integrity of joining liner 118 may be less than the structural integrity of the graft material of branching gate 116 because the primary function of joining liner 118 is a filler to reduce leakage paths, increase joint strength, and/or increase migration resistance whereas the graft material of branching gate 116 imparts structural integrity to branched stent graft 100.

Joining liner 118 may improve endurance reliability by creating a buffer between implant 140 and branching gate 116 to achieve leakage reductions, strength increases, and/or migration resistance. Joining liner 118 may also reduce packaging density using a lower cost material with less structural integrity as opposed to additional attachment mechanisms, stents, and/or sponges.

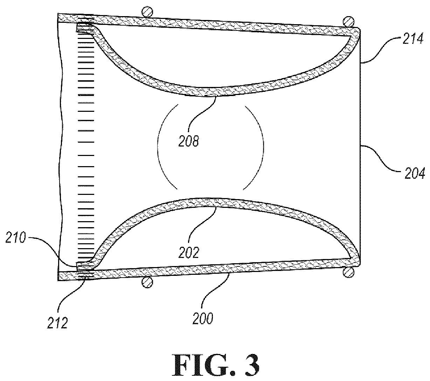

Figure 3 is a partial cross-sectional view of gate 200 of a branched stent graft where the material of gate 200 is folded inward back on itself to form joining liner 202 according to a third embodiment. Joining liner 202 continuously extends from gate 200 at opening 204. In this embodiment, gate 200 and joining liner 202 are formed of the same material. Non-limiting examples of the material include semi-permeable material or non-permeable material. A non-limiting example of a semi-permeable material is woven polyester terephthalate (PET). Non-limiting examples of non-permeable material include polyester terephthalate (PET), expanded polyester terephthalate (ePET), polytetrafluoroethylene (PTFE), and combinations thereof. Joining liner 202 may have a generally tubular shape with a bowed-in medial portion 208. As shown in Figure 3, proximal end 210 of joining liner 202 is joined to gate 200 by proximal joint 212. Materials used for the joints are set forth herein. As shown in Figure 3, a separate joint is not formed at distal end 214 of joining liner 202. Rather, distal end 214 of joining liner 202 may be crimped relative to gate 200 to reinforce the juncture between joining liner 202 and gate 200 without using a second separate joint. In one embodiment, distal end 214 may include stitching and/or suturing around at least a portion of or the entire circumference to provide reinforcement.

The joining liners of one or more embodiments may be suitable for complex stent graft systems used within branching vessel that utilize a branching lumen with reduced leakage, migrations, and fatigue by accommodating a wider range of non-uniform implants or short joint lengths. The joining liners of one or more embodiments may improve the joint connection reliability.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Aspects and embodiments of the invention may be defined by the following clauses.

Clause 1. An endovascular stent graft comprising:
a body including a gate having an internal surface;
a joining liner joined to the internal surface of the gate with a j oint and having a bunched state and a crumpled state; and
an implant at least partially disposed within the joining liner and having a radially compressed state and a radially expanded state, the implant in the radially expanded state exerting a radial force on the joining liner to maintain the joining liner in the crumpled state.

Clause 2. The endovascular stent graft of clause 1, wherein the gate is a coupling having a conical shape, and the joining liner and the implant have conical shapes complimentary to the conical shape of the gate.

Clause 3. The endovascular stent graft of any preceding clause, and in particular of clause 1, wherein the joining liner has a joining liner axial length, the gate has a gate axial length, and the bunched state includes an axially bunched state where the joining liner axial length is greater than the gate axial length.

Clause 4. The endovascular stent graft of any preceding clause, and in particular of clause 1, wherein the joining liner has a joining liner radial circumference, the gate has a gate radial circumference, and the bunched state includes a radially bunched state where the joining liner radial circumference is greater than the gate radial circumference at one or more locations along a longitudinal axis of the joining liner and gate.

Clause 5. The endovascular stent graft of any preceding clause, and in particular of clause 1, wherein the joining liner is formed of a semi-permeable material or a non-permeable material.

Clause 6. The endovascular stent graft of any preceding clause, and in particular of clause 1, wherein the body includes first and second branches, and the gate is located at a distal end of the first or second branch of the body.

Clause 7. The endovascular stent graft of any preceding clause, and in particular of clause 1, wherein the gate is formed of a first material and the joining liner is formed of a second material.

Clause 8. The endovascular stent graft of clause 7, wherein the first material has a material property different than the second material.

Clause 9. The endovascular stent graft of clause 8, wherein the material property is flexibility and the flexibility of the second material is greater than the first material.

Clause 10. The endovascular stent graft of clause 8, wherein the material property is strength and the strength of the first material is greater than the second material.

Clause 11. The endovascular stent graft of any preceding clause, and in particular of clause 1, wherein the gate and joining liner are formed of a continuous graft material.

Clause 12. The endovascular stent graft of clause 11, wherein the joining liner is formed from a first piece of the continuous graft material folded inwardly into the gate formed of a second piece of the continuous graft material.

Clause 13. An endovascular stent graft comprising:
a body including a gate having an internal surface;
a joining liner joined to the internal surface of the gate with a distal joint and a proximal joint, at least one of the distal and proximal joints extending around at least one of distal and proximal circumferences, respectively, of the gate; and
an implant at least partially disposed within the joining liner and having a radially compressed state and a radially expanded state, the implant in the radially expanded state contacting the joining liner.

Clause 14. The endovascular stent graft of clause 13, wherein the at least one of the distal and proximal joints extend continuously around the at least one of the distal and proximal circumferences, respectively, of the gate.

Clause 15. The endovascular stent graft of clause 13 or 14, wherein the distal and proximal joints extend continuously around the distal and proximal circumferences, respectively, of the gate to form a seal between the joining liner and the gate.

Clause 16. The endovascular stent graft of any one of clauses 13 to 15, and in particular of clause 13, wherein the body includes a graft material and a stent joined to the graft material with a body joint, and the at least one of the distal and proximal joints includes a portion of the body joint.

Clause 17. The endovascular stent graft of any one of clauses 13 to 16, and in particular of clause 13, wherein the body includes a graft material and a stent, and the at least one of the distal and proximal joints includes a melted and solidified portion of the graft material and the joining liner.

Clause 18. A method for joining an implant with a gate of an endovascular stent graft, the method comprising:
inserting the implant in a radially compressed state into the gate having an internal joining liner having a bunched state and a crumpled state; and
translating the implant from the radially compressed state into a radially expanded state to exert a radial force on the joining liner to maintain the internal joining liner in the crumpled state and to form a joint between the implant and the joining liner of the gate.

Clause 19. The method of clause 18, wherein the internal joining liner is in the bunched state during the inserting step.

Clause 20. The method of clause 18 or 19, wherein the internal joining liner contacts an internal surface of the gate when the internal joining liner is in the crumpled state.

Further disclosed herein is an endovascular stent graft. The endovascular stent graft includes a body including a gate having an internal surface, a joining liner joined to the internal surface of the gate with a joint and having a bunched state and a crumpled state, and an implant at least partially disposed within the joining liner and having a radially compressed state and a radially expanded state. The implant in the radially expanded state exerts a radial force on the joining liner to maintain the joining liner in the crumpled state.

## Claims

1. An endovascular stent graft comprising:
a body including a gate having an internal surface;
a joining liner joined to the internal surface of the gate with a joint and having a bunched state and a crumpled state; and
an implant at least partially disposed within the joining liner and having a radially compressed state and a radially expanded state, the implant in the radially expanded state exerting a radial force on the joining liner to maintain the joining liner in the crumpled state.

2. The endovascular stent graft of claim 1, wherein the gate is a coupling having a conical shape, and the joining liner and the implant have conical shapes complimentary to the conical shape of the gate.

3. The endovascular stent graft of any preceding claim, wherein the joining liner has a joining liner axial length, the gate has a gate axial length, and the bunched state includes an axially bunched state where the joining liner axial length is greater than the gate axial length.

4. The endovascular stent graft of any preceding claim, wherein the joining liner has a joining liner radial circumference, the gate has a gate radial circumference, and the bunched state includes a radially bunched state where the joining liner radial circumference is greater than the gate radial circumference at one or more locations along a longitudinal axis of the joining liner and gate.

5. The endovascular stent graft of any preceding claim, wherein the joining liner is formed of a semi-permeable material or a non-permeable material.

6. The endovascular stent graft of any preceding claim, wherein the body includes first and second branches, and the gate is located at a distal end of the first or second branch of the body.

7. The endovascular stent graft of any preceding claim, wherein the gate is formed of a first material and the joining liner is formed of a second material.

8. The endovascular stent graft of claim 7, wherein the first material has a material property different than the second material.

9. The endovascular stent graft of claim 8, wherein the material property is flexibility and the flexibility of the second material is greater than the first material; and/or wherein the material property is strength and the strength of the first material is greater than the second material.

10. The endovascular stent graft of any preceding claim, wherein the gate and joining liner are formed of a continuous graft material; and optionally wherein the joining liner is formed from a first piece of the continuous graft material folded inwardly into the gate formed of a second piece of the continuous graft material.

11. An endovascular stent graft comprising:
a body including a gate having an internal surface;
a joining liner joined to the internal surface of the gate with a distal joint and a proximal joint, at least one of the distal and proximal joints extending around at least one of distal and proximal circumferences, respectively, of the gate; and
an implant at least partially disposed within the joining liner and having a radially compressed state and a radially expanded state, the implant in the radially expanded state contacting the joining liner.

12. The endovascular stent graft of claim 11, wherein the at least one of the distal and proximal joints extend continuously around the at least one of the distal and proximal circumferences, respectively, of the gate.

13. The endovascular stent graft of claim 11 or 12, wherein the distal and proximal joints extend continuously around the distal and proximal circumferences, respectively, of the gate to form a seal between the joining liner and the gate.

14. The endovascular stent graft of any one of claims 11 to 13, wherein the body includes a graft material and a stent joined to the graft material with a body joint, and the at least one of the distal and proximal joints includes a portion of the body j oint; and/or wherein the body includes a graft material and a stent, and the at least one of the distal and proximal joints includes a melted and solidified portion of the graft material and the joining liner.

15. A method for joining an implant with a gate of an endovascular stent graft, the method comprising:
inserting the implant in a radially compressed state into the gate having an internal joining liner having a bunched state and a crumpled state; and
translating the implant from the radially compressed state into a radially expanded state to exert a radial force on the joining liner to maintain the internal joining liner in the crumpled state and to form a joint between the implant and the joining liner of the gate.
